# EUROPÄISCHE PATENTSCHRIFT

(11) **EP 0 915 816 B1**
(45) Veröffentlichungstag und Bekanntmachung des Hinweises auf die Patenterteilung: **25.10.2000**
(21) Anmeldenummer: 97933671.6
(22) Anmeldetag: 14.07.1997
(51) Int. Cl.: C07C 29/32, C07C 29/44, C07C 33/03, C07C 33/035

(54) **VERFAHREN ZUR HERSTELLUNG VON ALKOHOLEN UND/ODER ALDEHYDEN AUS OLEFINEN**
PROCESS FOR PREPARING ALCOHOLS AND/OR ALDEHYDES FROM OLEFINS
PROCEDE DE PREPARATION D'ALCOOLS ET/OU D'ALDEHYDES A PARTIR D'OLEFINES

(30) Priorität: 20.07.1996 DE 19629369; 11.10.1996 DE 19642278
(43) Veröffentlichungstag der Anmeldung: 19.05.1999
(73) Patentinhaber: BASF AKTIENGESELLSCHAFT, 67056 Ludwigshafen (DE)
(72) Erfinder: SCHWAB, Peter, D-67098 Bad Dürkheim (DE); HÖHN, Arthur, D-67281 Kirchheim (DE); PACIELLO, Rocco, D-67098 Bad Dürkheim (DE)
(86) Internationale Anmeldenummer: EP9703742
(87) Internationale Veröffentlichungsnummer: WO9803456

(56) Entgegenhaltungen:
- WO-A-96/04289
- P. SCHWAB, ET AL.: "Synthesis and applications of RuCl2(=CHR')(PR3)2: Influence of the alkyliden moiety on metathesis activity" JOURNAL OF THE AMERICAN CHEMICAL SOCIETY, Bd. 118, Nr. 1, 10.Januar 1996, WASHINGTON, DC, US, Seiten 100-110, XP002043841 in der Anmeldung erwähnt
- J.C. MOL, ET AL.: "Heterogeneous metathesis of alkenes having functional groups" JOURNAL OF THE CHEMICAL SOCIETY, CHEMICAL COMMUNICATIONS, Nr. 7, 5.April 1979, LETCHWORTH, GB, Seiten 330-331, XP002043945 in der Anmeldung erwähnt

## Beschreibung

Die vorliegende Erfindung betrifft ein Verfahren zur Herstellung von Alkoholen und/oder Aldehyden aus Olefinen durch Kreuzmetathese von Olefinen mit Alkoholen, die im Molekül mindestens eine C=C-Doppelbindung enthalten, an Katalysatoren der allgemeinen Formel (I)

X¹X²L¹L²Ru = CR¹R² (I)

in der
- R¹, R²: Wasserstoff oder einen organischen Rest oder einen Silylrest;
- X¹, X²: anionische Liganden;
- L¹, L²: neutrale Elektronendonor-Liganden
bedeuten; und gegebenenfalls anschließende Hydrierung und/oder Isomerisierung.

Alkohole sind wertvolle Lösungsmittel oder Zwischenprodukte in organischen Synthesen, zum Beispiel zur Herstellung von Phthalsäureestern, Acrylsäureestern oder Sulfonaten. Die Aldehyde dienen zum Beispiel als Zwischenprodukte zur Herstellung der entsprechenden Alkohole oder Säuren.

Eine allgemein bekannte und technisch verbreitete Reaktion von Olefinen ist die katalytische Olefinmetathese. Einen allgemeinen Überblick über die Olefinmetathese geben z.B. K. Weissermel und H.-J. Arpe in "Industrielle Organische Chemie", 4. Auflage, 1994, VCH Verlagsgesellschaft Weinheim, auf den Seiten 94 bis 98.

Die Olefinmetathese entspricht formal dem Tausch der Alkylidengruppen zweier Olefinmoleküle. Der Reaktionsmechanismus wird üblicherweise als metallkatalysierter Bruch der Doppelbindungen in den ursprünglichen Olefinen und ihre anschließende Neuformierung zu den Produkten formuliert. Die Reaktion ist reversibel. Ein allgemeines formales Reaktionsschema, das den Tausch der als R^{A}, R^{B}, ..R^{H} bezeichneten Substituenten verdeutlicht, ist ohne Berücksichtigung der tatsächlichen cis/trans-Stereochemie nachfolgend wiedergegeben:

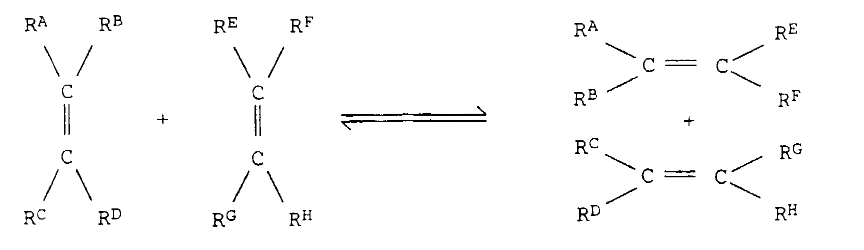

Im allgemeinen unterscheidet man zwischen der Selbstmetathese, bei der ein Olefin in ein Gemisch zweier Olefine unterschiedlicher Molmasse übergeht, zum Beispiel die Umwandlung zweier Propenmoleküle in ein Ethen- und ein 2-Butenmolekül, und der Kreuzmetathese, bei der zwei unterschiedliche Olefine in zwei neue Olefine übergehen, zum Beispiel die Umwandlung von Propen und 1-Buten zu Ethen und Penten.

Metathesereaktionen von funktionalisierten Olefinen, also solchen, die außer Kohlenstoff und Wasserstoff noch aus weiteren Elementen aufgebaut sind, sind im Gegensatz zu Metathesereaktionen von reinen Kohlenwasserstoff-Olefinen nur in sehr untergeordnetem Maße beschrieben. Die häufigsten Beispiele betreffen Metathesereaktionen ungesättigter Ether, Ester und Halogenide. So beschreiben zum Beispiel J. C. Mol und E. F. G. Woerlee in J. Chem. Soc., Chem. Commun., 1979, 330 - 331, die Selbstmetathese von Ethern und Formylestern ungesättiger Alkohole unter Abspaltung von Ethylen an einem zinnpromotierten Rheniumoxid-Katalysator auf einem Aluminiumoxidträger. US-A-5 342 985 lehrt die Selbstmetathese von Ethern und Estern ungesättigter Alkohole an speziellen Organorheniumoxiden.

In GB-A-2 131 429 werden die dort allgemein als "dismutation" bezeichneten Selbst- oder Kreuzmetathesen von funktionalisierten Olefinen beschrieben. Im Einklang mit dem gesamten sonstigen Stand der Technik werden dort laut Seite 1, Zeilen 49 bis 52, solche Sauerstoffsubstituenten, die keine Hydroxigruppe darstellen, bevorzugt. Die prinzipielle Eignung hydroxisubstituierter Olefine für Metathesereaktionen ist dieser Schrift nicht zu entnehmen. In ω-Position hydroxisubstituierte α-Olefine wie 4-Penten-1-ol, bei denen die Hydroxigruppe weiter von der Doppelbindung entfernt ist als der Allylposition entspricht, sind gemäß P. Schwab, R. H. Grubbs und J. W. Ziller, J. Am. Chem. Soc. 118, (1996), 100 - 110, mit einem speziellen Rutheniumkatalysator metathetisierbar. Derartige α-Olefine mit ω-ständiger Hydroxigruppe zeigen jedoch nicht die von Olefinen mit einer allylständigen Hydroxigruppe bekannte hohe Reaktivität und insbesondere nicht deren Allyl-Isomerisierungsneigung.

Bei einer Metathese eines α-Olefins mit einem Hydroxialken, bei dem der Sauerstoffsubstituent weiter von der Doppelbindung entfernt ist als der Allylposition entspricht, werden mindestens zwei Kohlenstoffatome an das ursprüngliche α-Olefin addiert. Auf Basis der technisch üblichen vorhandenen α-Olefine mit einer bestimmten Anzahl von Kohlenstoffatomen ist es durch Metathesereaktionen mit derartigen Hydroxialkenen also nicht möglich, die Alkohole oder Aldehyde mit einem Kohlenstoffgerüst, welches aus dem ursprünglichen Kohlenstoffgerüst des α-Olefins durch Addition genau eines Kohlenstoffatoms hervorgeht, zu gewinnen. Gerade Alkohole und Aldehyde, die durch Einbau nur eines C-Atoms erhalten werden, sind aber technisch interessant. Sie werden großtechnisch durch die sogenannte Oxo-Synthese, bei der α-Olefine mit einem Gemisch aus Kohlenmonoxid und Wasserstoff zu Aldehyden umgesetzt werden, erhalten. Dieses Verfahren der Hydroformylierung ist aber insofern nachteilig, als nur α-Olefine umgesetzt werden können und nach Hydrierung ausschließlich primäre Alkohole zugänglich sind. Darüber hinaus ist das Verhältnis von n-Produkten zu den unerwünschten iso-Produkten unbefriedigend. Allylalkohol ist durch Hydroformylierung nicht zugänglich, sondern muß auf einem mindestens zweistufigem Weg aus Propen hergestellt werden.

Der vorliegenden Erfindung lag somit die Aufgabe zugrunde, ein Verfahren zur Herstellung von Alkoholen und/oder Aldehyden aus α-Olefinen zu finden, das es ermöglicht, diese Produkte mit hoher Selektivität unter weitestgehender Vermeidung der Entstehung von iso-Produkten auf einfache Weise herzustellen. Darüber hinaus sollte dieses Verfahren industriell anstelle der üblicherweise verwendeten Oxo-Technologie eingesetzt werden können. Durch seine Anwendung sollten also aus den industriell allgemein vorhandenen Olefin-Einsatzstoffen die allgemein benötigten Alkohole und/oder Aldehyde hergestellt werden können. Dazu ist es notwendig, daß das Kohlenstoffgerüst des entstehenden Alkohols und/oder Aldehyds aus dem Kohlenstoffgerüst des verwendeten Olefins durch Erweiterung um genau ein Kohlenstoffatom hervorgeht. Das Verfahren sollte zur Erhöhung der Flexibilität darüber hinaus auch die Herstellung sekundärer und tertiärer Alkohole ermöglichen und sollte es ermöglichen, die industriell benötigten n-Alkohole auch aus anderen Einsatzstoffen als linearen α-Olefinen herstellen zu können. Weiterhin sollte das Verfahren einen einfachen Weg zur Herstellung von Allylalkohol eröffnen.

Dementsprechend wurde ein Verfahren zur Herstellung von Alkoholen und/oder Aldehyden aus Olefinen durch Kreuzmetathese von Olefinen mit Alkoholen, die im Molekül mindestens eine C=C-Doppelbindung enthalten, an Katalysatoren der allgemeinen Formel (I)

X¹X²L¹L²Ru = CR¹R² (I)

in der
- R¹, R²: Wasserstoff oder einen organischen Rest oder einen Silylrest;
- X¹, X²: anionische Liganden;
- L¹, L²: neutrale Elektronendonor-Liganden
bedeuten; und gegebenenfalls anschließende Hydrierung und/oder Isomerisierung gefunden, das dadurch gekennzeichnet ist, daß man Alkohole mit mindestens einer Doppelbindung im Molekül verwendet, in denen mindestens eine Hydroxigruppe in Allylstellung zu einer C=C-Doppelbindung steht.

Der Erfindung liegt die überraschende Erkenntnis zugrunde, daß die Rutheniumverbindungen der allgemeinen Formel (I) nicht nur gegen Hydroxigruppen stabil sind, sondern darüber hinaus auch Allylalkohole, also Olefine mit einer allylständigen Hydroxigruppe, die üblicherweise hochreaktiv sind und sehr leicht isomerisieren, an diesen Katalysatoren metathesiert werden können.

Als Ausgangsstoffe für das erfindungsgemäße Verfahren kommen Olefine der allgemeinen Formel (II):

R³R⁴C = CR⁵R⁶ (II)

mit im allgemeinen 3 bis 50 Kohlenstoffatomen, im besonderen Olefine mit 3 bis 30 Kohlenstoffatomen und bevorzugt Olefine mit 3 bis 20 Kohlenstoffatomen in Betracht. Die Reste R³, R⁴, R⁵ und R⁶ können unabhängig voneinander Wasserstoff oder ein aliphatischer, cycloaliphatischer, aromatischer, araliphatischer oder heteroaromatischer Rest, zum Beispiel Alkyl, Alkenyl, Alkinyl, Cycloalkyl, Cycloalkenyl, Aryl wie zum Beispiel Phenyl, Alkaryl oder Aralkyl sein. Die Reste können mit weiteren organischen Resten substituiert sein und auch Heteroatome, zum Beispiel in Form von Alkoxisubstituenten, Estergruppen, Amino- oder Alkylaminofunktionen umfassen. Die Heteroatome wie Sauerstoff, Schwefel und Stickstoff können auch Teil eines aromatischen oder cyclischen Rests sein. R³ kann mit R⁴ und/oder R⁵ kann mit R⁶ auch Teile eines Ringsystems, zum Beispiel mit 4 bis 16 Kohlenstoffatomen, darstellen, das R³, R⁴ und das an diese beiden Reste gebundene Kohlenstoffatom der Doppelbindung oder R⁵, R⁶ und das an diese beiden Reste gebundene Kohlenstoffatom der Doppelbindung umfaßt. Auch mono- oder polycyclische Olefine, also solche, bei denen R³ mit R⁵ oder R⁶ und/oder R⁴ mit R⁵ oder R⁶ und jeweils den Kohlenstoffatomen der Doppelbindung ein Ringsystem bildet und gegebenenfalls auch R³ mit R⁴ und/oder R⁵ mit R⁶ ebenfalls Teile von diese Reste und gegebenenfalls auch die entsprechenden Kohlenstoffatome der Doppelbindung umfassenden Ringsystemen sind, können unter Öffnung aller derer Ringe, welche die der Metathese unterworfene Doppelbindung enthalten, in Alkohole überführt werden.

In bevorzugter Weise werden im erfindungsgemäßen Verfahren monosubstituierte Olefine mit 3 bis 20 Kohlenstoffatomen und mindestens einer endständigen C=C-Doppelbindung eingesetzt. Bei diesen Olefinen sind R⁴, R⁵ und R⁶ Wasserstoff. R³ ist ein organischer Rest mit mindestens einem Kohlenstoffatom. Beispiele für im erfindungsgemäßen Verfahren bevorzugt verwendete α-Olefine sind diejenigen, in denen R³ ein linearer gesättigter Alkylrest mit einem bis 18 Kohlenstoffatomen wie Methyl, Ethyl, Propyl, Butyl, Pentyl, Hexyl, Heptyl, Octyl, Nonyl oder Decyl ist. R³ kann ebenso ein gesättigter cyclischer Alkylrest mit drei bis 18 Kohlenstoffatomen wie beispielsweise Cyclopentyl, Cyclohexyl oder Cycloheptyl sein. R³ kann auch ein verzweigter gesättigter Alkylrest wie 2-Propyl, 2-Butyl, 2-Methyl-1-propyl, 1,1-Dimethylethyl oder alle verzweigten isomeren Pentyl-, Hexyl-, Heptyl-, Octyl-, Nonyl- oder Decylreste sein. R³ kann auch ein ungesättigter Rest mit einer oder mehreren Doppel- und/oder Dreifachbindungen, der aus den oben genannten gesättigten Resten durch die formale Entfernung von mindestens zwei an benachbarten Kohlenstoffatomen befindlichen Wasserstoffatomen hervorgeht, sein, beispielsweise Vinyl, Propenyl, Butenyl, 1-Prop-2-enyl, 1-But-2-enyl oder 1-But-3-enyl. R³ kann ebenso ein aromatischer Rest sein, beispielsweise gegebenenfalls substituiertes Phenyl oder 1- oder 2-Naphthyl.

In ebenfalls bevorzugter Weise werden im erfindungsgemäßen Verfahren Cycloolefine mit 4 bis 16 Kohlenstoffatomen eingesetzt. In diesen Olefinen bildet R³ mit R⁵ oder R⁶ und/oder R⁴ mit R⁵ oder R⁶ und jeweils den Kohlenstoffatomen der Doppelbindung ein Ringsystem. Dieses Ringsystem kann außer der zu metathesierenden Doppelbindung oder den zu metathesierenden Doppelbindungen noch weitere Doppel- oder Dreifachbindungen oder auch Heteroatome wie Sauerstoff, Schwefel oder Stickstoff enthalten. Das Cycloolefin kann weiterhin mit inerten Resten substituiert sein. Beispiele für im erfindungsgemäßen Verfahren verwendbare Cycloolefine sind Cyclopenten, Cyclohepten, Cycloocten, Cyclooctadien, Cyclododecen, Cyclododecadien, Cyclododecatrien, dimeres Cyclopentadien, Norbornen oder Norbornadien.

Die im erfindungsgemäßen Verfahren verwendeten Alkohole mit mindestens einer Doppelbindung im Molekül haben mindestens eine Hydroxigruppe in Allylstellung zu einer Doppelbindung, wie in der allgemeinen Formel (III) verdeutlicht wird. Sie werden im folgenden allgemein als allylische Alkohole bezeichnet.

R⁷R⁸C = CR⁹ - C(OH)R¹⁰R¹¹ (III)

Die Reste R⁷, R⁸, R⁹, R¹⁰ und R¹¹ können unabhängig voneinander Wasserstoff oder ein aliphatischer, cycloaliphatischer, aromatischer, araliphatischer oder heteroaromatischer Rest, zum Beispiel Alkyl, Alkenyl, Alkinyl, Cycloalkyl, Cycloalkenyl, Aryl wie zum Beispiel Phenyl, Alkaryl oder Aralkyl sein. Die Reste können mit weiteren organischen Resten substituiert sein und auch Heteroatome, zum Beispiel in Form von Alkoxisubstituenten, Estergruppen, Amino- oder Alkylaminofunktionen umfassen. Die Heteroatome wie Sauerstoff, Schwefel und Stickstoff können auch Teil eines aromatischen oder cyclischen Rests sein. Die Reste können miteinander auch verknüpft sein und Teile eines Ringsystems, zum Beispiel mit 4 bis 12 Kohlenstoffatomen, darstellen.

Wenn im erfindungsgemäßen Verfahren ein allylischer Alkohol eingesetzt wird, dessen Reste R¹⁰ und/oder R¹¹ nicht Wasserstoff, sondern zum Beispiel eine oder zwei Alkyl- oder Arylgruppen, Alkenyl- oder Alkinylgruppen oder sonstige Substituenten darstellen, so entstehen sekundäre oder tertiäre Alkohole. Diese sekundären oder tertiären Alkohole sind mit dem Verfahren der Oxo-Synthese nicht herstellbar. Es ist ein zusätzlicher Vorteil des erfindungsgemäßen Verfahrens, sekundäre und tertiäre Alkohole erzeugen zu können.

Aufgrund des hohen industriellen Bedarfs an primären Alkoholen werden R¹⁰ und R¹¹ in den meisten Anwendungsfällen jedoch Wasserstoff sein.

R⁷, R⁸ und R⁹ können unabhängig voneinander beispielsweise Wasserstoff oder lineare gesättigte Alkylreste mit einem bis 10 Kohlenstoffatomen wie Methyl, Ethyl, Propyl, Butyl, Pentyl, Hexyl, Heptyl, Octyl, Nonyl oder Decyl sein. Sie können weiterhin gesättigte cyclische Alkylreste mit drei bis 10 Kohlenstoffatomen wie beispielsweise Cyclopentyl, Cyclohexyl oder Cycloheptyl sein. Sie können auch verzweigte gesättigte Alkylreste wie 2-Propyl, 2-Butyl, 2-Methyl-1-propyl, 1,1-Dimethylethyl oder alle verzweigten isomeren Pentyl-, Hexyl-, Heptyl-, Octyl-, Nonyl- oder Decylreste sein. Sie können darüber hinaus ungesättigte Reste mit einer oder mehreren Doppel- und/oder Dreifachbindungen, die aus den oben genannten gesättigten Resten durch die formale Entfernung von mindestens zwei an benachbarten Kohlenstoffatomen befindlichen Wasserstoffatomen hervorgehen, sein, beispielsweise Vinyl, Propenyl, Butenyl, 1-Prop-2-enyl, 1-But-2-enyl oder 1-But-3-enyl. Sie können ebenso aromatische Reste sein, beispielsweise gegebenenfalls substituiertes Phenyl oder 1- oder 2-Naphthyl. Alle genannten Reste können ebenso mit inerten Gruppen substituiert sein und/oder Heteroatome enthalten. Sie können auch über Heteroatome, zum Beispiel Sauerstoff-, Stickstoff- oder Schwefelatome an das C-Atom des allylischen Alkohols gebunden sein, etwa in Form von Alkoxi- oder Alkylthiogruppen mit einem bis 10 Kohlenstoffatomen.

Beispiele für technisch in sehr großen Mengen verfügbare und im erfindungsgemäßen Verfahren bevorzugt eingesetzte derartige Alkohole sind Prop-2-en-1-ol (Allylalkohol) und But-2-en-1,4-diol (Butendiol).

Die Alkohole können auch als Gemisch von mindestens zwei Einzelverbindungen verwendet werden, zum Beispiel als Gemisch von Allylalkohol und Butendiol.

In einer bevorzugten Ausführungsform der Erfindung wird Ethylen als Olefin eingesetzt und mit Butendiol zu Allylalkohol umgesetzt.

Bei der Metathese von acyclischen Olefinen mit allylischen Alkoholen entsteht neben einem neuen allylischen Alkohol ein Olefin-Koppelprodukt. Bei der Metathese von α-Olefinen mit Allylalkohol entsteht neben dem gewünschten Metatheseprodukt als Koppelprodukt Ethylen. Das Koppelprodukt, zum Beispiel Ethylen, kann im erfindungsgemäßen Verfahren wiedergewonnen und zum Beispiel in anderen Verfahren als Rohstoff eingesetzt werden. Bei der Metathese von α-Olefinen mit Butendiol entsteht im ersten Schritt neben dem gewünschten Metatheseprodukt als Koppelprodukt Allylalkohol. Dieser reagiert in einem zweiten Schritt mit dem α-Olefin unter Abspaltung des letztendlichen Koppelprodukts Ethylen. Wenn längerkettige allylische Alkohole, deren Reste R⁷ und/oder R⁸ nicht Wasserstoff sind, und/oder Olefine, deren Doppelbindung nicht endständig ist, eingesetzt werden, so entstehen als Koppelprodukte höhere Olefine. Wenn Gemische von Alkoholen, die mindestens einen höheren Allylalkohol enthalten, und/oder Gemische von Olefinen, die mindestens ein Olefin, dessen Doppelbindung nicht endständig ist, enthalten, eingesetzt werden, so entstehen Gemische von Koppelprodukten und/oder Produkten, die mit entsprechenden bekannten Stofftrennungsverfahren, zum Beispiel Destillation, aufgearbeitet werden können.

Bei der Metathese von cyclischen Olefinen mit allylischen Alkoholen wird der Ring, der die olefinische Doppelbindung enthält, geöffnet. Aus cycloolefinen kann so durch Metathese mit allylischen Alkoholen ein offenkettiger Alkohol erzeugt werden, dessen Kohlenstoffgerüst mindestens drei Kohlenstoffatome mehr umfaßt als das des eingesetzten Cycloolefins. Wird ein Cycloolefin erfindungsgemäß mit Butendiol umgesetzt, so entsteht ein α,ω-Dialkohol. Das erfindungsgemäße Verfahren eröffnet damit einen neuen Weg zur Herstellung von linearen Alkoholen auf Basis von cycloolefinen statt auf der Basis von α-Olefinen und schafft so eine höhere Flexibilität bei der Herstellung von Alkoholen.

Bei der Metathese von Ethylen mit Butendiol zu Allylalkohol entsteht naturgemäß nur Allylalkohol und kein Koppelprodukt. Eine Metathese von Ethylen mit Allylalkohol führt naturgemäß nicht zur Synthese eines neuen, nicht eingesetzten, Produkts.

Als Katalysatoren werden im erfindungsgemäßen Verfahren die in WO-A-93/20111 offengelegten Ruthenium-Alkylidenverbindungen, die von A. W. Stumpf, E. Saive, A. Demonceau und A. F. Noels in J. Chem. Soc., Chem. Commun. 1995, 1127 - 1128 beschriebenen Katalysatorsysteme auf Rutheniumbasis oder die von Schwab, Grubbs und Ziller, 1. c., bekannten Rutheniumverbindungen der allgemeinen Formel I eingesetzt.

X¹X²L¹L²Ru = CR¹R² (I)

In dieser Formel bedeuten R¹ und R² unabhängig voneinander Wasserstoff oder organische Reste oder Silylreste.

Als organische Reste geeignet sind zum Beispiel Alkyl-, Cycloalkyl- oder Arylreste mit einem bis 20 Kohlenstoffatomen, Alkenyl- oder Alkinylreste mit 2 bis 20 Kohlenstoffatomen, Carboxilatreste mit einem bis 20 Kohlenstoffatomen, Alkoxi- oder Aryloxireste mit einem bis 20 Kohlenstoffatomen, Alkenyloxi- oder Alkinyloxireste mit 2 bis 20 Kohlenstoffatomen, Alkoxicarbonylreste mit 2 bis 20 Kohlenstoffatomen, Alkylthioreste mit einem bis 20 Kohlenstoffatomen oder Alkylsulfonyl- oder Alkylsulfinylreste mit einem bis 20 Kohlenstoffatomen, die wiederum substituiert sein können.

Als Silylreste sind mit organischen Resten substituierte Silylreste geeignet. Als organische Substituenten sind beispielsweise Alkyl-, Cycloalkyl- oder Arylreste mit einem bis 20 Kohlenstoffatomen, Alkoxi- oder Aryloxireste mit einem bis 20 Kohlenstoffatomen oder Dialkyl- oder Diaryl- oder Alkylarylaminoreste geeignet.

Bevorzugte Katalysatoren sind solche Rutheniumverbindungen der allgemeinen Formel (I), bei denen einer der beiden Reste R¹ und R² Wasserstoff ist. Besonders bevorzugt werden solche Rutheniumverbindungen der allgemeinen Formel (I), bei denen einer der beiden Reste R¹ und R² Wasserstoff und der andere entweder eine Arylgruppe, zum Beispiel eine Phenylgruppe, oder eine Alkenylgruppe, zum Beispiel eine in 2-Position mit einem organischen, bevorzugterweise aromatischen Rest wie einer Phenylgruppe, substituierte 1-Ethenylgruppe, oder eine Silylgruppe, zum Beispiel eine Trialkyl- oder Triarylsilylgruppe, wie die Trimethylsilylgruppe oder die Triphenylsilylgruppe, ist.

Die Arylgruppen, zum Beispiel Phenylgruppen, die im organischen Rest oder im Alkenylrest oder im Silylrest vorhanden sein können, können auch wiederum substituiert sein, zum Beispiel mit einer oder mehreren Alkyl-, Cycloalkyl-, Aryl-, Alkoxi- Alkylthio- oder Aminoresten mit je einem bis 6 C-Atomen. Beispiele für geeignete Substituenten sind die Methyl-, Ethyl-, n-Propyl-, iso-Propyl-, n-Butyl-, iso-Butyl-, tert.-Butyl, Cyclohexyl-, Cyclopentyl-, Phenyl-, Methoxi-, Ethoxi-, Methylthio-, Ethylthio-, Amino-, Methylamino- oder Dimethylaminogruppen.

X¹ und X² bedeuten anionische Liganden, zum Beispiel unabhängig voneinander Halogene wie Fluor, Chlor, Brom oder Iod, Wasserstoff, Alkyl-, Cycloalkyl- oder Arylreste mit einem bis 20 Kohlenstoffatomen, Alkoxi- oder Aryloxireste mit einem bis 20 Kohlenstoffatomen, Alkyl- oder Aryldiketonate mit 3 bis 20 Kohlenstoffatomen, Carboxilatreste mit einem bis 20 Kohlenstoffatomen, Alkyl- oder Arylsulfonate mit einem bis 20 Kohlenstoffatomen, Alkylthioreste mit einem bis 20 Kohlenstoffatomen oder Alkylsulfonyl- oder Alkylsulfinylreste mit einem bis 20 Kohlenstoffatomen. Die genannten Reste können wiederum substituiert sein, zum Beispiel durch Halogen, Alkyl- oder Alkoxireste. Aryl steht vorzugsweise für Phenyl oder Naphthyl.

L¹ und L² bedeuten neutrale Elektronendonor-Liganden. L¹ und L² können zum Beispiel unabhängig voneinander Phosphan oder alkyl-, cycloalkyl- oder arylsubstituierte Phosphane bedeuten, Phosphite, Phosphinite oder Phosphonite, Arsan, Stiban, Ammoniak, oder alkyl-, cycloalkyl- oder arylsubstituierte Arsane, Stibane, Ether, aliphatische und aromatische Amine, Amide, Mercaptane, Thioether, Sulfoxide, Kohlenmonoxid, Nitrosyl oder π-Elektronendonoren wie aromatische Verbindungen, zum Beispiel mit organischen Resten substituiertes Benzol bedeuten. Als organische Reste eines Benzol-Liganden sind inerte organische Reste geeignet, zum Beispiel Alkyl-, Alkenyl- oder Alkoxigruppen. Ein Beispiel für einen derartigen als neutralen Liganden geeigneten aromatischen π-Elektronendonor ist zum Beispiel Paramethylcumol.

Diese Liganden X¹, X², L¹ und L² können vier einzelne Liganden sein, es können aber auch mehrzähnige anionische Liganden oder mehrzähnige neutrale Liganden verwendet werden. Genauso können mehrzähnige Liganden verwendet werden, die sowohl anionische als auch neutrale komplexierende Funktionen aufweisen, wie zum Beispiel Cyclopentadienyl- Indenyl- oder Fluorenylreste.

In bevorzugter Form bedeuten X¹ und X² Halogenidionen. In besonders bevorzugter Form bedeuten sowohl X¹ als auch X² Chlorid. In bevorzugter Form bedeuten L¹ und L² alkyl-, cycloalkyl- oder aryl-, zum Beispiel phenylsubstituierte Phosphanliganden. In besonders bevorzugter Form bedeuten L¹ und L² cycloalkylsubstituierte Phosphanliganden, zum Beispiel Tris(cyclohexyl)phosphan oder Tris(cyclopentyl)phosphan.

Der erfindungsgemäß zu verwendende Katalysator kann getrennt von der Metathesereaktion hergestellt werden und dem Reaktionsgemisch der Metathese zugesetzt werden. Es ist jedoch genauso möglich, in aus seinen Ausgangsstoffen in situ im Reaktionsgemisch der Metathesereaktion erst herzustellen.

Prinzipiell kann das erfindungsgemäße Verfahren kontinuierlich oder diskontinuierlich durchgeführt werden. Das erfindungsgemäße Verfahren wird im allgemeinen durch Inkontaktbringen des Alkohols mit dem Olefin und dem Katalysator in einem Reaktor und gleichzeitiger oder anschließender Aufarbeitung des Reaktionsgemisches durchgeführt. Unter gleichzeitiger Aufarbeitung ist zum Beispiel die Abtrennung mindestens eines Teils von mindestens einem Produkt oder Koppelprodukt aus dem Reaktionsgemisch, während die Reaktion durchgeführt wird, zu verstehen.

Metathesereaktionen sind Gleichgewichtsreaktionen, so daß sich in Abhängigkeit des im Einzelfall verwendeten Olefins, Alkohols und Katalysators je nach den gewählten Reaktionsbedingungen wie Druck, Temperatur und Konzentrationen der Einsatzstoffe eine bestimmte Lage des Gleichgewichts einstellt. Um eine möglichst wirtschaftliche Verfahrensführung zu erreichen, ist es also im allgemeinen zweckmäßig, die Reaktionsbedingungen so zu wählen, daß das Gleichgewicht möglichst weit auf der Seite der gewünschten Produkte liegt. Weiterhin ist es, wenn die Lage des Gleichgewichts nicht bereits von vornherein besonders vorteilhaft ist, im allgemeinen wirtschaftlich zweckmäßig, durch möglichst rasche Entfernung der Produkte und/oder Koppelprodukte aus dem Reaktionsgemisch das Gleichgewicht auf die Produktseite zu verschieben und so für möglichst vollständigen Umsatz und hohe Raumzeitausbeute zu sorgen. Diese Verschiebung des Gleichgewichts kann, wenn nötig, zum Beispiel durch Entfernung des bei der Metathese neben dem gewünschten Alkohol-Produkt entstehenden Olefin-Koppelprodukts erfolgen. Genauso könnte das gewünschte Produkt selbst aus dem Reaktionsgemisch entfernt werden, wenn die Abtrennung des Produkts technisch oder wirtschaftlich vorteilhafter ist als die des Koppelprodukts. Es ist ebenfalls möglich, Produkt und Koppelprodukt gleichzeitig aus dem Reaktionsgemisch zu entfernen und anschließend voneinander zu trennen. Die Verschiebung des Gleichgewichts kann aber genauso durch Zugabe eines Ausgangsstoffs im Überschuß oder durch eine Kombination der beiden Methoden erfolgen.

Bei der Reaktion eines α-Olefins mit Allylalkohol und/oder Butendiol wird das Koppelprodukt Ethylen gebildet, das aufgrund seiner hohen Flüchtigkeit leicht, zum Beispiel durch Destillation, aus dem Reaktionsraum abzutrennen ist. Dies gilt auch für die höheren Olefin-Koppelprodukte, die bei der Verwendung von Olefinen, deren Doppelbindung nicht endständig ist, und/oder bei der Verwendung von längerkettigen Allylalkoholen entstehen, solange ihre Flüchtigkeit höher ist als die des anderen Produkts oder der anderen Produkte. Wenn die Produkte deutlich flüchtiger ist als die Koppelprodukte, können anstelle der Koppelprodukte auch die Produkte aus dem Reaktionsraum entfernt und so dem Gleichgewicht entzogen werden. Wenn die Flüchtigkeit von Koppelprodukten und Produkten ähnlich groß ist, kann zum Beispiel durch Destillation ein Gemisch der Koppelprodukte und Produkte aus dem Reaktionsraum entfernt werden, das anschließend aufgetrennt wird, zum Beispiel durch Feindestillation, azeotrope Destillation, gegebenenfalls unter Zusatz von mindestens einem Hilfsstoff oder andere bekannte Methoden der Stofftrennung.

Das Verfahren kann beispielsweise in Rührkesselreaktoren, Schlaufenreaktoren, Rührkesselkaskaden oder in Rohrreaktoren durchgeführt werden. Diesen Reaktoren sind zur Abtrennung von Produkten, Koppelprodukten und/oder Nebenprodukten in der Anlage zur Durchführung des erfindungsgemäßen Verfahrens Trenneinrichtungen wie zum Beispiel Destillationskolonnen, Phasenabscheider, Membrantrennvorrichtungen, Absorptionsvorrichtungen oder gleichwertige Apparate beigeordnet.

Bei einer diskontinuierlichen Durchführung des erfindungsgemäßen Verfahrens wird eine Mischung aus dem Olefin-Einsatzstoff, dem Alkohol-Einsatzstoff und dem Katalysator oder einer Lösung des Katalysators in einem Reaktor zur Reaktion gebracht. Dabei wird vorteilhafterweise der Katalysator oder die Lösung des Katalysators zu einer Mischung des verwendeten Olefin-Einsatzstoffs und des Alkohol-Einsatzstoffs und gegebenenfalls eines Lösungsmittels gegeben. Sobald ein zufriedenstellender Umsatz erreicht ist, wird das Reaktionsgemisch aufgearbeitet und die Produkte werden isoliert. Alternativ kann, wenn die Lage des Gleichgewichts unbefriedigend ist, nach oder bereits während der Zugabe des Katalysators oder der Katalysatorlösung mit der Entfernung der Produkte und/oder Koppelprodukte aus dem Reaktionsraum und damit aus dem Gleichgewicht begonnen werden. Dies kann beispielsweise durch Destillation eines kontinuierlich aus dem Reaktor entnommenen Stoffstroms und Rückführung der unumgesetzten Einsatzstoffe in den Reaktionsraum geschehen. Der im Destillationssumpf zurückbleibende Katalysator kann wieder in einem neuen Ansatz verwendet werden.

Bei einer kontinuierlichen Durchführung des erfindungsgemäßen Verfahrens werden der Katalysator oder eine Lösung des Katalysators, der Olefin-Einsatzstoff und der Alkohol-Einsatzstoff und kontinuierlich in den Reaktionsraum eingebracht, in dem sich weiterhin ein Lösungsmittel befinden kann. Es kann vorteilhaft sein, die Komponenten zumindest teilweise als ein Gemisch von mindestens zwei Komponenten, zum Beispiel ein Gemisch des Olefins und des Alkohols, statt der reinen Komponenten zuzugeben, zum Beispiel dann, wenn aus der Aufarbeitung der Reaktionsprodukte des erfindungsgemäßen Verfahrens ein Gemisch unumgesetzter Einsatzstoffe oder aus anderen Quellen ein zur Verwendung im erfindungsgemäßen Verfahren geeignetes Stoffgemisch zur Verfügung steht. Aus dem Reaktionsraum werden kontinuierlich Koppelprodukte und Produkte entfernt. Dies kann beispielsweise durch Destillation eines kontinuierlich aus dem Reaktor entnommenen Stoffstroms und Rückführung der unumgesetzten Einsatzstoffe und gegebenenfalls des Lösungsmittels in die Reaktion geschehen. Der im Destillationssumpf zurückbleibende Katalysator kann ebenfalls in die Reaktion zurückgeführt werden. Wenn nötig, zum Beispiel bei auftretender Desaktivierung, kann der Katalysator auch vor einer Rückführung wiederaufgearbeitet oder zumindest teilweise durch frischen ersetzt werden.

Die Abtrennung der Produkte und/oder der Koppelprodukte kann statt durch Destillation zum Beispiel auch durch Membrantrennverfahren, Absorptionsverfahren oder durch Kombinationen der Methoden erfolgen.

Im Normalfall entstehen im erfindungsgemäßen Verfahren außer den gewünschten Produkten und den anfallenden Koppelprodukten auch Nebenprodukte. Diese Nebenprodukte sind zum Beispiel die durch die Selbstmetathese des verwendeten Olefins entstehenden höheren Olefine. Wenn beispielsweise ein α-Olefin verwendet wird, so kann dieses in einer Metathesereaktion mit sich selbst unter Abspaltung von Ethen in ein symmetrisches Olefin mit der Doppelbindung in der Mitte seines Kohlenstoffgerüsts übergehen. Diese höheren Olefine sind zwar ebenso zur Metathese mit den einzusetzenden allylischen Alkoholen befähigt, zeigen dabei aber im allgemeinen eine gegenüber α-Olefinen reduzierte Reaktionsgeschwindigkeit, so daß, wenn die Metathese von α-Olefinen durchgeführt werden soll, im Interesse einer wirtschaftlich optimalen Raumzeitausbeute ihre Bildung unerwünscht ist und durch möglichst rasche Entfernung der gewünschten Produkte aus dem Gleichgewicht vermieden wird. Die Nebenprodukte können gemeinsam mit den gewünschten Produkten und den Koppelprodukten aus dem Reaktionsgemisch entfernt werden, zum Beispiel durch Destillation. Sie können jedoch auch durch ein gesondertes Trennverfahren für die Nebenprodukte entfernt werden, zum Beispiel durch Destillation eines eigens zu diesem Zweck aus dem Reaktionsraum entnommenen Teilstroms und Rückführung aller anderen Bestandteile außer den Nebenprodukten in den Reaktionsraum. Bei kontinuierlicher Durchführung des Verfahrens ist ihre Entfernung zu empfehlen, da ihre Konzentration im Reaktionsgemisch sonst stetig zunimmt.

Der Katalysator kann als Feststoff verwendet werden oder vorzugsweise in einem inerten organischen Lösungsmittel, zum Beispiel unter Reaktionsbedingungen flüssigen aliphatischen Kohlenwasserstoffen mit 4 bis 20 Kohlenstoffatomen wie Butan, Pentan, Hexan oder Heptan, Chlorkohlenwasserstoffen wie Methylenchlorid, Chloroform oder Tetrachlorkohlenstoff oder aromatischen Kohlenwasserstoffen wie Benzol, Toluol oder Xylolen gelöst sein. Bevorzugt ist die Verwendung von Methylenchlorid oder Toluol. Die Reaktion selbst kann unter Verwendung eines Lösungsmittels, neben dem gegebenenfalls verwendeten Lösungsmittel für den Katalysator, oder zur Erhöhung der Raumzeitausbeute im allgemeinen vorzugsweise lösungsmittelfrei, abgesehen vom gegebenenfalls für den Katalysator verwendeten Lösungsmittel, durchgeführt werden. Wenn ein Lösungsmittel verwendet wird, kann ein anderes oder vorzugsweise das gleiche Lösungsmittel wie bei der Lösung des Katalysators verwendet werden. Wird als Alkohol 1,4-But-2-en-diol in höheren Konzentrationen verwendet, so kann es vorteilhaft sein, einen Löslichkeitsvermittler zuzugeben. Als Löslichkeitsvermittler kann zum Beispiel Aceton und/oder Ethylenglykoldibutylether verwendet werden.

Die zur Durchführung der Reaktion geeignete Temperatur ist im allgemeinen niedriger als 200°C. Zur Vermeidung von Nebenreaktionen ist es meistens vorteilhaft, die Reaktion unterhalb von 120°C vorzunehmen. Bevorzugterweise wird das erfindungsgemäße Verfahren unterhalb von 80°C durchgeführt. Nach unten ist der im erfindungsgemäßen Verfahren anwendbare Temperaturbereich nur durch das Gebot wirtschaftlich befriedigender Raumzeitausbeuten begrenzt. Im allgemeinen ist es sinnvoll, Temperaturen oberhalb - 20°C, bevorzugt oberhalb 0°C und in besonders bevorzugter Weise oberhalb von + 20°C zu wählen.

Der im erfindungsgemäßen Verfahren anzuwendende Reaktionsdruck ist im allgemeinen kein kritischer Parameter. Er kann zum Beispiel bis 325 bar Überdruck betragen, vorzugsweise wird ein Überdruck nicht höher als 10 bar und im besonderen ein Überdruck nicht höher als 2 bar eingestellt. Im allgemeinen wird der absolute Reaktionsdruck oberhalb von 20 mbar liegen, vorzugsweise oberhalb von 0,1 bar und im besonderen oberhalb von 0,5 bar.

Das molare Verhältnis des eingesetzten Olefins zum eingesetzten Alkohol kann prinzipiell in sehr weiten Grenzen variiert werden. Es ist aufgrund der zunehmenden Bildung von Produkten der Selbstmetathese von Olefin oder Alkohol vorteilhaft, dieses Verhältnis im Bereich von 100 : 1 bis 1 : 100 einzustellen. Meistens wird es vorteilhaft sein, es im Bereich von 10 : 1 bis 1 : 10 einzustellen. Bevorzugterweise liegt dieses Verhältnis im Bereich von 2 : 1 bis 1 : 2 und in besonders bevorzugter Weise im Bereich von 1,5 : 1 bis 1 : 1,5. Das molare Verhältnis von eingesetztem Olefin zum Katalysator liegt im allgemeinen im Bereich von 10 : 1 bis 20 000 : 1, vorzugsweise im Bereich von 50 : 1 bis 8 000 : 1 und in besonders bevorzugter Weise im Bereich von 100 : 1 bis 5 000 : 1.

In der Regel ist die Umsetzung nach einer Minute bis fünf Stunden beendet.

Die Produkte der Metathesereaktion zwischen einem Olefin und einem allylischen Alkohol sind naturgemäß wiederum ein Olefin und ein allylischer Alkohol, oder, wenn ein cyclisches Olefin verwendet wurde, ist das Produkt wiederum ein allylischer Alkohol mit einer bezogen auf die neue Allyleinheit ω-ständigen Doppelbindung.

Sofern im erfindungsgemäßen Verfahren ein Olefin entsteht, wird es vom produzierten allylischen Alkohol abgetrennt und als Koppelprodukt gewonnen. Das Olefin-Koppelprodukt kann anschließend in jedem anderen Verfahren, in dem Olefine als Einsatzstoff Verwendung finden, zum Beispiel Polymerisation, Oxidation, thermisches oder katalytisches Cracken oder Metathesereaktionen eingesetzt werden.

Der im Verlauf des erfindungsgemäßen Verfahrens in der Stufe der Metathesereaktion hergestellte allylische Alkohol hat, ohne daß hier der möglichen cis/trans-Isomerie in irgendeiner Form Rechnung getragen wird, die allgemeine Formel (IV):

R³R⁴C = CR⁹ - C(OH)R¹⁰R¹¹ (IV),

in der die Substituenten R³, R⁴, R⁹, R¹⁰ und R¹¹ die oben genannten Bedeutungen haben.

Diese Alkohole können prinzipiell als solche verwendet werden. Im Normalfall werden sie aber hydriert werden, um die allylische Doppelbindung in eine Einfachbindung umzuwandeln. Dies kann nach jedem bekannten Hydrierverfahren, mit dem C=C-Doppelbindungen in C-C-Einfachbindungen umgewandelt werden können ohne Hydroxigruppen zu reduzieren, geschehen. Wenn gewünscht, können gleichzeitig in diesem Hydrierschritt weitere Doppelbindungen, die unter Umständen in den Resten R³, R⁴, R⁹, R¹⁰ und R¹¹ enthalten sind, beispielsweise bei Verwendung cyclischer Olefine, ebenfalls mit hydriert werden. Beispielsweise können Alkohole der allgemeinen Formel (IV) in Gegenwart heterogener Katalysatoren, die Metalle der 8. Nebengruppe enthalten, mit Wasserstoff umgesetzt werden. Geeignete Katalysatoren sind zum Beispiel Platin oder Palladium auf inerten Trägern wie porösem Siliciumdioxid oder porösem Aluminiumoxid.

Wenn mindestens einer der beiden Reste R¹⁰ und R¹¹ im allylischen Alkohol der Formel (IV) Wasserstoff ist, können auch durch Umlagerung der allylischen Doppelbindung und die sich automatisch anschließende Keto/Enol-Tautomerisierung aus dem allylischen Alkohol Aldehyde oder Ketone hergestellt werden. Aldehyde entstehen, wenn beide Reste R¹⁰ und R¹¹ Wasserstoff sind, wenn nur einer der beiden Wasserstoff ist, so entstehen Ketone. Diese Umlagerung wird zum Beispiel durch bestimmte Rutheniumverbindungen oder metallisches Ruthenium auf einem inerten Aluminiumoxidträger katalysiert. Verfahren, die eine derartige Umlagerung unter Rutheniumkatalyse nutzen und die auch im erfindungsgemäßen Verfahren zur Weiterverarbeitung des hergestellten allylischen Alkohols zu Aldehyden oder Ketonen einsetzbar sind, sind beispielsweise aus den Schriften US-A-4 117 016, WO-A-91/03449 oder WO-A-95/19334 bekannt.

Die derart aus einem verwendeten α-Olefin durch Umsetzung mit Allylalkohol als Endprodukt entstehenden Alkohole oder Aldehyde entsprechen den Produkten, die aus einer Oxo-Synthese und gegebenenfalls einer nachfolgenden Hydrierung unter Einsatz desselben Olefins hervorgehen, mit dem Unterschied, daß bei einer Vorgehensweise nach dem erfindungsgemäßen Verfahren der bei der Oxo-Synthese zwangsweise anfallende Anteil an unerwünschten iso-Aldehyden und iso-Alkoholen nicht anfällt. Weiterhin können die gewünschten Aldehyde und Alkohole im Gegensatz zur Oxo-Synthese auch aus cyclischen Olefinen gewonnen werden. Darüber hinaus können im Gegensatz zur Oxo-Synthese mit dem erfindungsgemäßen Verfahren bei Einsatz allylischer Alkohole, von deren Reste R¹⁰ und R¹¹ mindestens einer nicht Wasserstoff sind, auch sekundäre und tertiäre Alkohole oder Ketone hergestellt werden.

### Beispiele

### Beispiele 1 bis 6

In einem Schlenkrohr wurde ein Gemisch aus 5 ml Olefin und einer äquimolaren Menge Allylalkohol vorgelegt und bei Raumtemperatur mit einer Lösung von RuCl₂(=CHPh)(PCy₃)₂ (Ph bedeutet eine Phenylgruppe, Cy ein Cyclohexylgruppe) in CH₂Cl₂ versetzt. Die Molverhältnisse von Olefin, Alkohol und Katalysator betrugen 100 : 100 : 1. Binnen weniger Minuten beobachtete man in allen Fällen eine charakteristische Farbänderung der Lösung von violett über weinrot nach orange. Entstandenes Ethen konnte über einen Blasenzähler entweichen. Nach einer Reaktionszeit von 2 h wurden Proben entnommen und gaschromatographisch analysiert. Die verwendeten Olefine, die erzielten Umsätze an Olefin und die gemessenen Selektivitäten zu Alkohol aus der Kreuzmetathese und zu Nebenprodukten aus der Selbstmetathese des Olefins sind in der folgenden Tabelle 1 wiedergegeben.

**Tabelle 1**

| Bsp. Nr. | Olefin | Umsatz [Mol-%] | Selektivität zum Alkohol [Mol-%] | Selektivität zu Nebenprodukten [Mol-%] |
|---|---|---|---|---|
| 1 | 1-Hexen | 16 % | 74 % Heptenol | 25 % Decen |
| 2 | 1-Octen | 12 % | 72 % Nonenol | 28 % Tetradecen |
| 3 | 1-Nonen | 13 % | 72 % Decenol | 26 % Hexadecen |
| 4 | 1-Decen | 12 % | 72 % Undecenol | 27 % Octadecen |
| 5 | 1-Undecen | 12 % | 71 % Dodecenol | 27 % Eicosen |
| 6 | 1-Dodecen | 11 % | 74 % Tridecenol | 26 % Doeicosen |

### Beispiel 7

In einem Schlenkrohr wurde ein äquimolares Gemisch aus 25 ml Cyclopenten und 19.5 ml Allylalkohol (jeweils 0.29 Mol) vorgelegt und bei Raumtemperatur mit einer Lösung von 239 mg (0.29 mMol) RuCl₂(=CHPh) (PCy₃)₂ in CH₂Cl₂ versetzt. Neben einer heftigen Gasentwicklung (Ethen konnte über einen Blasenzähler entweichen) beobachtete man binnen weniger Minuten die o. g. charakteristische Farbänderung der Lösung. Nach einer Reaktionszeit von 30 min wurde eine Probe entnommen und gaschromatographisch analysiert. Der Umsatz an Cyclopenten betrug 83 Mol-% und die Selektivitäten zu den einzelnen Reaktionsprodukten betrugen in Mol-%:

| | |
|---|---|
| 2,7-Octadien-1-ol | 59; |
| 2,7,12-Tridecatrien-1-ol | 27; |
| 2,7,12,17-Octadecatetraen-1-ol | 7; |
| But-2-en-1,4-diol | 6; |
| 1,6-Heptadien | 1. |

### Beispiel 8

In einem Schlenkrohr wurde ein äquimolares Gemisch aus 25 ml cis-Cycloocten und 15 ml Allylalkohol (jeweils 0.19 Mol) vorgelegt und bei Raumtemperatur mit einer Lösung von 156 mg (0.19 mMol) RuCl₂(=CHPh) (PCy₃)₂ in CH₂Cl₂ versetzt. Neben einer allmählichen Gasentwicklung (Ethen konnte über einen Blasenzähler entweichen) beobachtete man binnen weniger Minuten die o. g. charakteristische Farbänderung der Lösung. Nach einer Reaktionszeit von 2 h wurde eine Probe entnommen und gaschromatographisch analysiert. Der Umsatz an Cycloocten betrug 33 Mol-% und die Selektivitäten zu den einzelnen Reaktionsprodukten betrugen in Mol-%:

| | |
|---|---|
| 2,10-Undecadienol-1-ol | 76; |
| 2,10,18-Nonadecatrien-1-ol | 4; |
| But-2-en-1,4-diol | 19; |
| 1,9-Decadien | 1. |

### Beispiel 9

In einem Schlenkrohr wurde ein äquimolares Gemisch aus 25 ml 1,5-Cyclooctadien und 13.9 ml Allylalkohol (jeweils 0.2 Mol) vorgelegt und bei Raumtemperatur mit einer Lösung von 250 mg (0.3 mMol) RuCl₂(=CHPh) (Pcy₃)₂ in CH₂Cl₂ versetzt. Neben einer heftigen Gasentwicklung (Ethen konnte über einen Blasenzähler entweichen) beobachtete man binnen weniger Minuten die o. g. charakteristische Farbänderung der Lösung. Nach einer Reaktionszeit von 2 h wurde eine Probe entnommen und gaschromatographisch analysiert. Der Umsatz an Cyclooctadien betrug 48 Mol-% und die Selektivitäten zu den einzelnen Reaktionsprodukten betrugen in Mol-%:

| | |
|---|---|
| 2,6,10-Undecatrien-1-ol | 65; |
| 2,6,10,14,18-Nonadecapentaen-1-ol | 5; |
| But-2-en-1,4-diol | 8; |
| 1,5,9-Decatrien | 22. |

### Beispiel 10

In einem Schlenkrohr wurde ein äquimolares Gemisch aus 25.0 ml Cyclopenten und 23.9 ml But-2-en-1,4-diol (jeweils 0.29 Mol) in 5 ml Ethylenglykoldibutylether vorgelegt und bei Raumtemperatur mit einer Lösung von 239 mg (0.29 mMol) RuCl₂(=CHPh) (PCy₃)₂ in CH₂Cl₂ versetzt. Man beobachtete binnen weniger Minuten die o. g. charakteristische Farbänderung der Lösung. Nach einer Reaktionszeit von 30 min wurde eine Probe entnommen und gaschromatographisch analysiert. Der Umsatz an Cyclopenten betrug 24 Mol-% und die Selektivitäten zu den einzelnen Reaktionsprodukten betrugen in Mol-%:

| | |
|---|---|
| 2,7-Nonadien-1,6-diol | 64; |
| 2,7,12-Tetradectrien-1,13-diol | 36. |

### Beispiel 11

In einem Druckbehälter wurde 40.0 g Butendiol vorgelegt und bei Raumtemperatur mit einer Lösung von 400 mg RuCl₂ (=CHPh) (PCy₃)₂ (Ph bedeutet eine Phenylgruppe, Cy eine Cyclohexylgruppe) in 12 ml eines Gemisches aus gleichen Volumenteilen CH₂Cl₂ und N-Methylpyrrolidon versetzt. Anschließend wurde bei Raumtemperatur Ethylen bis zu einem Druck von 30 bar aufgepreßt, danach die Reaktionsmischung auf 50°C erwärmt und der Druck durch weitere Zufuhr von Ethylen auf 75 bar erhöht. Im Verlaufe von 2 Stunden wurde der im Verlauf der Reaktion sinkende Druck durch weitere Zugaben von Ethylen auf 75 bar gehalten. Anschließend wurde der Druckbehälter entspannt und der Reaktionsaustrag gaschromatographisch analysiert. Die Auswertung des Gaschromatogramms nach Flächen-% ergab einen Butendiolumsatz von 58 % und eine Selektivität zu Allylalkohol von 87 %.

## Patentansprüche

1. Verfahren zur Herstellung von Alkoholen und/oder Aldehyden aus Olefinen durch Kreuzmetathese von Olefinen mit Alkoholen, die im Molekül mindestens eine C=C-Doppelbindung enthalten, an Katalysatoren der allgemeinen Formel (I)
X¹X²L¹L²Ru = CR¹R² (I)
in der
R¹, R² Wasserstoff oder einen organischen Rest oder einen Silylrest;
X¹, X² anionische Liganden;
L¹, L² neutrale Elektronendonor-Liganden
bedeuten; und gegebenenfalls anschließende Hydrierung und/ oder Isomerisierung, dadurch gekennzeichnet, daß man Alkohole mit mindestens einer Doppelbindung im Molekül verwendet, in denen mindestens eine Hydroxigruppe in Allylstellung zu einer C=C-Doppelbindung steht.

2. Verfahren nach Anspruch 1, dadurch gekennzeichnet, daß man ein monosubstituiertes Olefin mit mindestens einer endständigen C=C-Doppelbindung verwendet.

3. Verfahren nach Anspruch 2, dadurch gekennzeichnet, daß man ein lineares Olefin mit einer endständigen C=C-Doppelbindung verwendet.

4. Verfahren nach Anspruch 1, dadurch gekennzeichnet, daß man ein cyclisches Olefin mit mindestens einer C=C-Doppelbindung verwendet.

5. Verfahren nach Anspruch 4, dadurch gekennzeichnet, daß man ein monocyclisches Olefin mit einer C=C-Doppelbindung verwendet.

6. Verfahren nach Anspruch 1, dadurch gekennzeichnet, daß man als Alkohol mit mindestens einer Doppelbindung im Molekül Allylalkohol (Prop-2-en-1-ol) und/oder Butendiol (But-2-en-1,4-diol)verwendet.

7. Verfahren nach Anspruch 1, dadurch gekennzeichnet, daß man Allylalkohol durch Metathese von Ethylen mit But-2-en-1,4-diol herstellt.

8. Verfahren nach Anspruch 1, dadurch gekennzeichnet, daß man einen Metathesekatalysator der allgemeinen Formel (I) verwendet, in der X und X¹ Halogene, L und L¹ substituierte Phosphane, R¹ einen Arylrest und R² Wasserstoff darstellen.

9. Verfahren nach Anspruch 1, dadurch gekennzeichnet, daß man die Metathese bei Temperaturen von - 20 bis + 120°C durchführt.

10. Verfahren nach Anspruch 1, dadurch gekennzeichnet, daß das molare Verhältnis des eingesetzten Olefins zum eingesetzten Alkohol im Bereich von 100 : 1 bis 1 : 100 liegt.

11. Verfahren nach Anspruch 1, dadurch gekennzeichnet, daß man die Metathese bei einem Druck von 0,5 bis 325 bar durchführt.

## Claims

1. A process for preparing alcohols and/or aldehydes from olefins by cross-metathesis of olefins with alcohols whose molecule includes at least one C=C double bond over catalysts of the formula (I)
X¹X²L¹L²Ru = CR¹R² (I)
where
R¹ and R² are hydrogen or an organic radical or a silyl radical;
X¹ and X² are anionic ligands; and
L¹ and L² are neutral electron donor ligands;
with optional subsequent hydrogenation and/or isomerization, which comprises using alcohols having at least one double bond in the molecule, in which at least one hydroxyl is in the allyl position relative to a C=C double bond.

2. A process as claimed in claim 1, wherein a monosubstituted olefin having at least one terminal C=C double bond is used.

3. A process as claimed in claim 2, wherein a linear olefin having a terminal C=C double bond is used.

4. A process as claimed in claim 1, wherein a cyclic olefin having at least one C=C double bond is used.

5. A process as claimed in claim 4, wherein a monocyclic olefin having a C=C double bond is used.

6. A process as claimed in claim 1, wherein as alcohol having at least one double bond in the molecule use is made of allyl alcohol (2-propen-1-ol) and/or butenediol (2-butene-1,4-diol).

7. A process as claimed in claim 1, wherein allyl alcohol is prepared by metathesis of ethylene with 2-butene-1,4-diol.

8. A process as claimed in claim 1, wherein a metathesis catalyst of the formula (I) is used in which X and X¹ are halogens, L and L¹ are substituted phosphines, R¹ is aryl and R² is hydrogen.

9. A process as claimed in claim 1, wherein the metathesis is conducted at from -20 to +120°C.

10. A process as claimed in claim 1, wherein the molar ratio of the olefin employed to the alcohol employed is in the range from 100 : 1 to 1 : 100.

11. A process as claimed in claim 1, wherein the metathesis is conducted at a pressure of from 0.5 to 325 bar.

## Revendications

1. Procédé pour la préparation d'alcools et/ou d'aldéhydes d'oléfines par métathèse croisée d'oléfines avec des alcools, qui contiennent dans la molécule au moins une double liaison C=C, sur des catalyseurs de formule générale (I)
X¹X²L¹L²Ru = CR¹R² (I)
dans laquelle
R¹, R² représentent l'hydrogène ou un reste organique ou un reste silyle;
X¹, X² désignent des ligands anioniques;
L¹, L² représentent des ligands neutres, donneurs d'électrons;
et éventuellement hydrogénation et/ou isomérisation subséquente, caractérisé par le fait qu'on utilise des alcools ayant au moins une double liaison dans la molécule, dans lesquels au moins un groupe hydroxy est en position allylique d'une double liaison C=C.

2. Procédé selon la revendication 1, caractérisé par le fait qu'on utilise une oléfine monosubstituée ayant au moins une double liaison C=C terminale.

3. Procédé selon la revendication 2, caractérisé par le fait qu'on utilise une oléfine linéaire ayant une double liaison C=C terminale.

4. Procédé selon la revendication 1, caractérisé par le fait qu'on utilise une oléfine cyclique ayant au moins une double liaison C=C.

5. Procédé selon la revendication 4, caractérisé par le fait qu'on utilise une oléfine monocyclique ayant une double liaison C=C.

6. Procédé selon la revendication 1, caractérisé par le fait qu'on utilise comme alcool ayant au moins une double liaison dans la molécule de l'alcool allylique (prop-2-ène-1-ol) et/ou du butènediol (but-2-ène-1,4-diol).

7. Procédé selon la revendication 1, caractérisé par le fait qu'on prépare l'alcool allylique par métathèse d'éthylène avec du but-2-ène-1,4-diol.

8. Procédé selon la revendication 1, caractérisé par le fait qu'on utilise un catalyseur de métathèse de formule générale (I), dans laquelle X et X¹ représentent des halogènes, L et L¹ désignent des phosphanes substitués, R¹ représente un reste aryle et R² représente l'hydrogène.

9. Procédé selon la revendication 1, caractérisé par le fait qu'on effectue la métathèse à des températures de -20 à +120°C.

10. Procédé selon la revendication 1, caractérisé par le fait que le rapport molaire entre l'oléfine employée et l'alcool mis en oeuvre se situe dans l'intervalle de 100 : 1 à 1 : 100.

11. Procédé selon la revendication 1, caractérisé par le fait qu'on effectue la métathèse à une pression de 0,5 à 325 bar.
